(19) Europäisches Patentamt
European Patent Office
Office européen des brevets

(11) Publication number: **0 422 889 A2**

(12) # EUROPEAN PATENT APPLICATION

(21) Application number: **90311043.5**

(51) Int. Cl.[5]: **C07C 217/10**

(22) Date of filing: **09.10.90**

(30) Priority: **10.10.89 GB 8922795**
**05.06.90 GB 9012472**

(43) Date of publication of application:
**17.04.91 Bulletin 91/16**

(84) Designated Contracting States:
**AT BE CH DE DK ES FR GB GR IT LI LU NL SE**

(71) Applicant: **GLAXO GROUP LIMITED**
**Clarges House 6-12 Clarges Street**
**London W1Y 8DH(GB)**

(72) Inventor: **Evans, Brian**
**Glaxo Group Research Limited, Park Road**
**Ware, Hertfordshire, SG12 0DG(GB)**

(74) Representative: **Marchant, James Ian et al**
**Elkington and Fife Beacon House 113**
**Kingsway**
**London WC2B 6PP(GB)**

(54) **Phenethanolamine compounds.**

(57) The present invention provides the compound (R)-(-)-4-hydroxy-$\alpha^1$-[[[6-(4-phenylbutoxy)hexyl]amino]methyl]-1,3-benzenedimethanol or physiologically acceptable salts thereof, processes for its preparation, pharmaceutical formulations containing it, and its use in medicine.

EP 0 422 889 A2

# PHENETHANOLAMINE COMPOUND

This invention relates to an optically active phenethanolamine derivative having a stimulant action at $\beta_2$-adrenoreceptors, tc processes for its preparation, to pharmaceutical compositions containing it and to its use in medicine.

In our British Patent Specification No. 2140800 we have described a novel class of phenethanolamine derivatives, including the racemic mixture of the compound 4-hydroxy-$\alpha^1$-[[[6-(4-phenylbutoxy)hexyl]amino]-methyl]-1,3-benzenedimethanol (hereinafter referred to as "Compound A"), having stimulant activity at $\beta_2$-adrenoreceptors.

We now provide an optical isomer of Compound A.

Thus, according to one aspect of the present invention we provide the compound: (R)-(-)-4-hydroxy-$\alpha^1$-[[[6-(4-phenylbutoxy)hexyl]amino]methyl]-1,3-benzenedimethanol, and its physiologically acceptable salts.

The (R)-isomer of Compound A is provided substantially free of the corresponding (S)-isomer.

As used herein, the term "substantially free" means that the (R)-isomer has an enantiomeric excess of at least 90% preferably 96%, and in particular 98%.

As used herein, "enantiomeric excess" is an indication of the relative proportion of one enantiomer to the other, and is simply the percentage difference between the ratio of the two enantiomers. Thus, for example, a 75:25 ratio of enantiomers is equivalent to a 50% enantiomeric excess and a 95:5 ratio is equivalent to a 90% enantiomeric excess.

The compound according to the invention has a selective stimulant action at $\beta_2$-adrenoreceptors, which furthermore is of a particularly advantageous profile. The stimulant action may be demonstrated in the isolated trachea of the guinea-pig where the compound causes relaxation of contractions induced by $PGF_{2\alpha}$ or electrical stimulation. The compound also has a prolonged duration of action.

The compound according to the invention may be used in the therapy or prophylaxis of conditions susceptible to amelioration by a compound possessing selective stimulant action at $\beta_2$-adrenoreceptors, particularly of diseases associated with reversible airways obstruction such as asthma and chronic bronchitis. The compound according to the invention may also be used in the therapy or prophylaxis of inflammation, allergy or allergic reaction. Further examples of conditions which may be alleviated by administration of a compound possessing selective $\beta_2$-stimulant activity are inflammatory and allergic skin diseases, depression, premature labour, glaucoma and conditions in which there is an advantage in lowering gastric acidity, particularly in gastric and peptic ulceration.

In a further or alternative aspect of the invention, therefore, we provide the (R)-isomer of Compound A or a physiologically acceptable salt thereof for use in medicine, more particularly for use in the treatment of conditions which may be ameliorated by administration of a compound having a selective stimulant action at $\beta_2$-adrenoreceptors.

In another aspect, the invention further provides a method for the treatment of a condition subject to amelioration by a compound having selective stimulant activity at $\beta_2$-adrenoreceptors in a mammal, including a human, comprising administration of an effective amount of the (R)-isomer of Compound A or a physiologically acceptable salt thereof.

There is also provided in a further or alternative aspect use of the (R)-isomer of Compound A or a physiologically acceptable salt thereof, for the manufacture of a medicament for the treatment of a condition which may be ameliorated by a compound having selective $\beta_2$-adrenoreceptor stimulant activity.

It is to be understood that references herein to treatment may extend to prophylaxis as well as the treatment of established conditions.

Suitable physiologically acceptable salts of the compound of the invention include acid addition salts derived from inorganic and organic acids, such as hydrochlorides, hydrobromides, sulphates, phosphates, maleates, tartrates, citrates, benzoates, 4-methoxy-benzoates, 2- or 4-hydroxybenzoates, 4-chlorobenzoates, benzenesulphonates, p-toluenesulphonates, naphthalenesulphonates, methanesulphonates, sulphamates, ascorbates, salicylates, acetates, diphenylacetates, triphenylacetates, adipates, fumarates, succinates, lactates, glutarate, gluconates, tricarballylates, hydroxynaphthalenecarboxylates e.g. 1-hydroxy or 3-hydroxy-2-naphthalenecarboxylates, or oleates. The compound may also form salts with suitable bases. Examples of such salts are alkali metal (e.g. sodium and potassium) and alkaline earth metal (e.g. calcium or magnesium) salts, and salts with organic bases (e.g. triethylamine).

Salts having a very low solubility in water are particularly convenient where the compound is to be administered by inhalation or insufflation. Such salts include diphenylacetates, 4,4'-methylenebis 3-hydroxy-2-naphthalenecarboxylates and 1-hydroxy- and 3-hydroxy-2-naphthalenecarboxylates.

It is possible that the compound of the invention may be administered to a patient as the raw chemical,

but it is preferable to present the active ingredient as a pharmaceutical formulation.

The invention accordingly provides a pharmaceutical formulation comprising the (R)-isomer of Compound A or a physiologically acceptable salt thereof together with one or more physiologically acceptable carriers and, optionally, other therapeutic and/or prophylactic ingredients. The carrier(s) must be "acceptable" in the sense of being compatible with the other ingredients of the formulation and not deleterious to the recipient thereof.

The compound may be formulated in a form suitable for administration by inhalation or insufflation, or for oral, buccal, parenteral, topical (including nasal) or rectal administration. Administration by inhalation or insufflation is preferred.

For administration by inhalation the compound according to the invention are conveniently delivered in the form of an aerosol spray presentation from pressurised packs, with the use of a suitable propellant, such as dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation or insufflation, the compound according to the invention may take the form of a dry powder composition, for example a powder mix of the compound and a suitable powder base such as lactose or starch. The powder composition may be presented in unit dosage form in for example capsules or cartridges of e.g. gelatin, or blister packs from which the powder may be administered with the aid of an inhaler or insufflator.

For oral administration, the pharmaceutical composition may take the form of, for example, tablets, capsules, powders, solutions, syrups or suspensions prepared by conventional means with acceptable excipients.

For buccal administration the composition may take the form of tablets, drops or lozenges formulated in the conventional manner.

The compound of the invention may be formulated for parenteral administration by bolus injection or continuous infusion. Formulation for injection may be presented in unit dosage form in ampoules, or in multi-dose containers with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water, before use.

For topical administration the pharmaceutical composition may take the form of ointments, lotions or creams formulated in a conventional manner, with for example an aqueous or oily base, generally with the addition of suitable thickening agents and/or solvents. For nasal application, the composition may take the form of a spray, formulated for example as an aqueous solution or suspension or as an aerosol with the use of a suitable propellant.

The compound of the invention may also be formulated in rectal compositions such as suppositories or retention enemas, e.g. containing conventional suppository bases such as cocoa butter or other glyceride.

Where pharmaceutical compositions are described above for oral, buccal, rectal or topical administration, these may be presented in a conventional manner associated with controlled release forms.

A proposed daily dosage of active compound for the treatment of man is 0.005mg to 100mg, which may be conveniently administered in one or two doses. The precise dose employed will of course depend on the age and condition of the patient and on the route of administration. Thus a suitable dose for administration by inhalation is 0.005mg to 20mg, for oral administration is 0.02mg to 100mg, and for parenteral administration is 0.01mg to 2mg for administration by bolus injection and 0.01mg to 25mg for administration by infusion.

The compound of the invention may be prepared according to the general methods described in British Patent Specification No. 2140800 from starting materials having the desired stereochemical configuration.

The (R)-isomer of Compound A may conveniently be prepared by deprotection of a compound of formula (I):

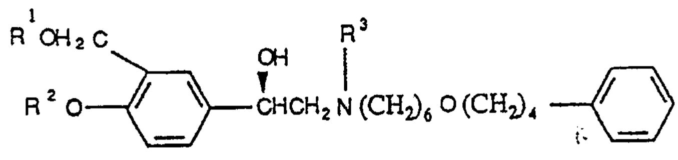

(I)

wherein $R^1$, $R^2$ and $R^3$ each independently represents a hydrogen atom or a protecting group with the

proviso that at least one of them represents a protecting group.

The protecting groups may be any conventional protecting groups, for example as described in "Protective Groups in Organic Synthesis" by Theodora Greene (John Wiley and Sons Inc. 1981). Examples of suitable hydroxyl protecting groups represented by $R^1$ and $R^2$ include aralkyl groups such as benzyl, diphenylmethyl or triphenylmethyl groups, acyl groups such as acetyl, and tetrahydropyranyl. Examples of suitable amino protecting groups represented by $R^3$ include aralkyl groups such as benzyl, $\alpha$-methylbenzyl, $\alpha$-hydroxymethylbenzyl, diphenylmethyl or triphenylmethyl groups and acyl groups such as acetyl, trichloroacetyl or trifluoroacetyl.

In a preferred embodiment of the process, $R^3$ is a protecting group which contains an asymmetric centre, for example $R^3$ may represent the group

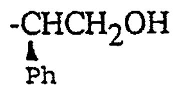 or 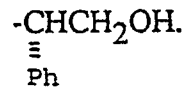

The deprotection to yield the (R)-isomer of Compound A may be effected using conventional techniques. Thus, for example, when $R^1$, $R^2$ and/or $R^3$ is a substituted or unsubstituted aralkyl group this may be cleaved by hydrogenolysis in the presence of a metal catalyst (e.g. palladium on charcoal). Tetrahydropyranyl groups may be cleaved by hydrolysis under acidic conditions. Acyl groups may be removed by hydrolysis with an acid such as mineral acid, e.g. hydrochloric acid, or a base such as sodium hydroxide or potassium carbonate, and a group such as trichloroacetyl may be removed by reduction with, for example, zinc and acetic acid.

Intermediates of formula (I) wherein $R^3$ is the group

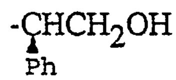

may be prepared by reduction of compounds of formula (IIR)

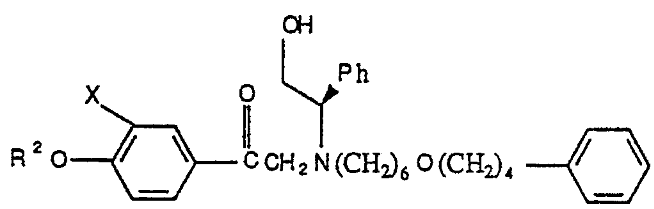

(IIR)

wherein X represents $-CH_2OR^1$ or $-CO_2R^4$, $R^1$ and $R^2$ are as defined for formula (I) and $R^4$ represents a straight or branched chain $C_{1-4}$alkyl group, using a hydride such as diborane or a complex metal hydride such as lithium aluminium hydride, sodium borohydride or preferably lithium borohydride in a suitable solvent, such as an ether, for example, 1,2-dimethoxyethane, preferably at elevated temperature.

Similarly, intermediates of formula (I) wherein $R^3$ is the group

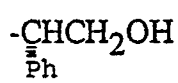

may be analogously prepared from compounds of formula (IIS)

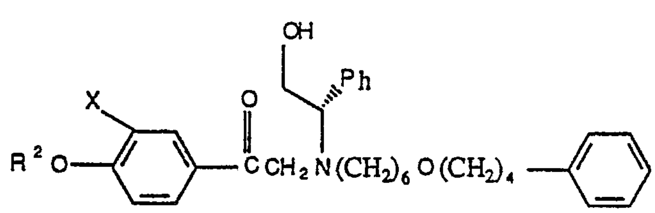

(IIS)

wherein X and $R^2$ are defined as above.

It will be appreciated that reduction of a compound of formula (IIR) or (IIS) will yield a mixture of two diastereoisomers the separation of which may therefore be effected at any time prior to the removal of the (R)- or (S)-2-hydroxy- 1 -phenylethyl group.

In a preferred embodiment of the process, the diastereoisomers are separated when X represents $-CH_2OR^1$, $R^3$ represents either an (R)- or an (S)-2-hydroxy-1-phenylethyl group and $R^1$ and $R^2$ each represent hydroxyl protecting groups.

In a further preferred embodiment of the process, the diastereoisomers are separated when X represents $-CO_2R^4$, $R^4$ represents $C_{1-4}$alkyl, $R^3$ represents an (R)- or (S)-2-hydroxy-1-phenylethyl group and $R^2$ represents a hydroxyl protecting group.

Compounds of formula (IIR) may be prepared by reaction of a compound of formula (III)

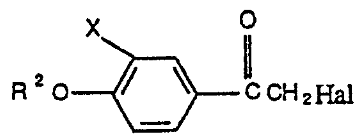

(III)

wherein X and $R^2$ are as defined above and Hal represents a halogen atom, for example a chlorine or iodine atom or preferably a bromine atom, with the compound of formula (IVR)

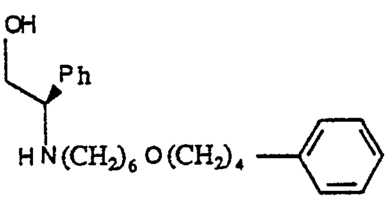

(IVR)

The reaction is effected in a suitable solvent, such as acetonitrile or an ether, for example 1,2-dimethoxyethane, preferably at elevated temperature and preferably in the presence of a base such as diisopropylethylamine, sodium carbonate or other acid scavenger such as propylene oxide.

Compounds of formula (IIS) may be analogously prepared from a compound of formula (III) and the compound of formula (IVS)

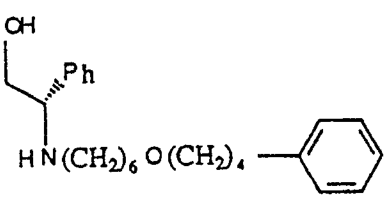

(IVR)

Intermediates of formula (IVR) may be prepared from a compound of formula (V)

$L(CH_2)_6O(CH_2)_4Ph$ (V)

wherein L represents a leaving group, for example a halogen atom such as chlorine, bromine or iodine, or a hydrocarbylsulphonyloxy group such as methanesulphonyloxy or p-toluenesulphonyloxy, by reaction with (R)-phenylglycinol. The reaction is preferably effected in the presence of a suitable acid scavenger, for example a base such as diisopropyethylamine conveniently in a solvent such as acetonitrile or an ether, e.g. tetrahydrofuran, a substituted amide, e.g. dimethylformamide or a chlorinated hydrocarbon, e.g. chloroform at a temperature between ambient and the reflux temperature of the solvent.

Intermediates of formula (IVS) may be prepared analogously from a compound of formula (V) and (S)-phenylglycinol.

The preparation of compounds of formulae (III) and (V) is described in British Patent Specification No. 2140800.

Intermediates of formula (I) for use in the deprotection process may be alternatively be prepared by reaction of a chiral epoxide of formula (VI)

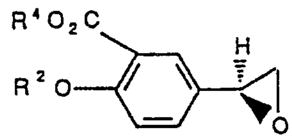

(VI)

(wherein $R^2$ and $R^4$ are as defined above) with an amine of formula (VII)

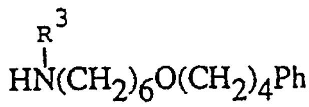

(VII)

(wherein $R^3$ is as defined in formula (I)) conveniently in a suitable solvent, such as an alcohol, for example methanol, at a temperature between ambient and the reflux temperature of the solvent, followed by reduction of the group $-CO_2R^4$ by conventional means, for example using a hydride reducing agent as described above.

The preparation of epoxides of formula (VI) is described in British Patent Specification No. 2140800.

The (R)-isomer of Compound A may also be obtained by resolution of racemic Compound A using conventional methods.

The term "resolution" is used herein in the conventional practical sense used in the art to include partial resolution, that is, the separation of a mixture of enantiomers of a compound (in any ratio) into two fractions, one of which is enriched in one enantiomer relative to the initial mixture.

Resolution of the mixture of (R)- and (S)-isomers of Compound A may be effected conventionally by derivatising the mixture with a chiral derivatising agent, to form a mixture of diastereomers of a derivative of Compound A. The components of the mixture may then be separated conventionally, for example by fractional crystallisation. Separation may be complete or partial. The derivatisation is preferably effected by reacting a mixture of the (R)- and (S)-isomers of Compound A with a suitable optically active acid. Suitable optically active acids include carboxylic and sulphonic organic acids, for example (+)- or (-)-tartaric acid and (+) or (-)-dibenzoyl tartaric acid.

The preferred process for the preparation of the (R)-isomer of Compound A is from the chiral intermediate of formula (I).

The (R)-isomer of Compound A, however prepared, may be converted to its acid addition salts by treatment with a suitable acid. Thus, for example, the (R)-isomer of Compound A may be converted to its 1-hydroxy-2-naphthoate salt by treatment with 1-hydroxy-2-naphthoic acid.

The following examples illustrate the invention.

Temperatures are in °C. Thin layer chromatography (T.l.c.) and flash column chromatography were carried out over silica and "dried" refers to drying using sodium sulphate.

## Example 1

### (R)-(-)-4-Hydroxy-$\alpha^1$-[[[6-(4-phenylbutoxy)hexyl]amino]methyl]-1,3-benzenedimethanol, 1-hydroxy-2-naphthoate (salt)

#### (i) (R)-1-[[6-(4-Phenylbutoxy)hexyl]amino]benzeneethanol

A mixture of 6-(4-phenylbutoxy)hexanol, methanesulphonate (32.8g) and (R)-phenylglycinol (15.0g) in dry acetonitrile (700ml), containing N,N-diisopropylethylamine (15.6g) and sodium iodide (16.5g) was heated to 75° for 20h. Water (1L) was added to the cooled mixture which was extracted with toluene (3 x 750ml). The organic phases were combined and solvent removed *in vacuo* . Purification of the residue by flash column chromatography eluting with dichloromethane-methanol-0.88 ammonia (300:8:1 → 150:8:1) gave the title compound as a yellow oil (23.8g).

T.l.c. (dichloromethane:ethanol:0.88 ammonia, 200:8:1) Rf 0.19.

(ii) [R-(R*,R*)]-$\alpha^1$-[[(2-Hydroxy-1-phenylethyl)[6-(4-phenylbutoxy)hexyl]amino]methyl]-4-(phenylmethoxy)-benzene- 1,3-dimethanol

A mixture of methyl 5-(bromoacetyl)-2-(phenylmethoxy)benzoate (7.26g), the product of part (i) (7.38g) and N,N-diisopropyl ethylamine (3.86g) in 1,2-dimethoxyethane (100ml) was heated under reflux for 4h. The cooled suspension was filtered and the solid discarded. To the filtrate was added lithium borohydride (1.74g) and the mixture was heated under reflux for 4h. 2N Hydrochloric acid (25ml) was cautiously added to the cooled solution followed by 2N sodium carbonate (30ml). The mixture was poured into ethyl acetate (250ml) and water (500ml). After separating the phases, the aqueous phase was extracted with ethyl acetate (100ml). The combined organic extracts were dried and evaporated under reduced pressure. The residue was chromatographed on silica gel (Sorbsil C60; 20-40$\mu$, 600g) using hexane-ethyl acetate-triethylamine [6:3:1) as eluant yielding the title compound as a pale yellow oil (4.8g).
T.l.c. (hexane-ethyl acetate-triethylamine [6:3:1]) eluted twice, Rf 0.3.

(iii) (R)-(-)-4-Hydroxy-$\alpha^1$-[[[6-(4-phenylbutoxy)hexyl]amino] methyl]-1,3-benzenedimethanol

A solution of the product of part (ii) (1.0g) in ethanol (50ml) containing 10% palladium on charcoal (50% wet paste, 0.5g) was stirred under an atmosphere of hydrogen until uptake ceased. The suspension was filtered through "hyflo" and the filtrate was evaporated under reduced pressure. The residue was chromatographed on silica gel (Art 7734; 30g) eluting with dichloromethane-methanol-ammonia [60:8:1] to give the title compound as a pale yellow oil (0.58g).T.l.c. (dichloromethane-methanol-ammonia [60:8:1]), Rf 0.27
Analysis Found: C,72.3; H,9.2; N,3.3.
$C_{25}H_{37}NO_4$ requires C,72.25; H,9.0; N,3.4%.

(iv) (R)-(-)-4-Hydroxy-$\alpha^1$-[[[6-(4-phenylbutoxy)hexyl]amino]methyl]- 1,3-benzenedimethanol 1-hydroxy-2-naphthoate salt (1:1)

A mixture of the product of part (iii) (0.53g) and 1-hydroxy-2- naphthoic acid (0.24g) was dissolved in methanol (10ml). The solution was evaporated under reduced pressure and the residue crystallised from isopropyl acetate (5ml) as an off-white powder (0.43g), m.p. 90-91°C.
T.l.c. (dichloromethane-methanol-ammonia [60:8:1]), Rf 0.27.
Analysis Found: C,71.1; H,7.6; N,2.2.
$C_{25}H_{37}NO_4.C_{11}H_8O_3.0.1H_2O$ requires : C,71.4; H,7.5; N,2.3%.
$(\alpha)_D^{20.5}$-20.1 (methanol, 0.005g/ml)
Water content 0.13% w/w.

Example 2

(S)-(+)-4-Hydroxy-$\alpha^1$-[[[6-(4-phenylbutoxy)hexyl]amino]methyl]-1,3-benzenedimethanol, 1-hydroxy-2-naphthoate salt

(i) (S)-1-[[6-(4-Phenylbutoxy)hexyl]amino]benzeneethanol

The title compound (5.35g) was prepared from 6-(4-phenylbutoxy) hexanol, methanesulphonate (6.5g) and (S)-phenylglycinol (3.00g) by the methcd of Example 1, part (i).
Analysis Found: C,79.2; H,10.0; N,4.1;
$C_{24}H_{35}NO_2$ requires : C,78.0; H,9.55; N,3.8%.

(ii) [S-(R*,R*)]-$\alpha^1$-[[(2-Hydroxy-1-phenylethyl)[6-(4-phenylbutoxy)hexyl]amino]methyl]-4-[(phenylmethoxy)-benzene-1,3-dimethanol

The title compound (2.45g) was prepared from methyl 5-bromoacetyl-2-(phenylmethoxy)benzoate (5.30g) and the product of part (i) (5.35g) by the method of Example 1, part (ii).
T.l.c. (hexane-ethylacetate-triethylamine [6:3:1]) Rf 0.25
Analysis Found: C,76.8; H,8.5; N,2.65;
$C_{40}H_{51}NO_5$ requires: C,76.8; H,8.2; N,2.2%

(iii) (S)-(+)-4-Hydroxy-α[1]-[[[6-(4-phenylbutoxy)hexyl]amino] methyl]-1,3-benzenedimethanol

The title compound (0.48g) was prepared from the product of part (ii) (1.07g) by the method of Example 1, part (iii) using 10% palladium oxide on charcoal as the hydrogenation catalyst.
T.l.c. (dichloromethane-methanol- 0.88 ammonia [60:8:1]) Rf 0.25.

(iv) (S)-(+)-4-Hydroxy-α[1]-[[[6-(4-phenylbutoxy)hexyl]amino]methyl]- 1,3-benzenedimethanol, 1-hydroxy-2-naphthoate salt (1:1)

The product of part (iii) (0.43g) was dissolved in tert-butylmethyl ether (3ml) and 1-hydroxy-2-naphthoic acid (200mg) in tert -butylmethyl ether (3ml) added to the solution. A gum was formed, the solvent removed in vacuo and the salt triturated with diethyl ether to give a white crystalline powder (0.49g) m.p. 90-92°.
Analysis Found: C,7 1.3; H,7.6; N,2.2.
$C_{25}H_{37}NO_4.C_{11}H_8O_3$ requires : C,71.6; H,7.6; N,2.3%.
$[\alpha]_D^{20.2}$ +20.18 (methanol, 0.010g/ml)

Examples 3 and 4 illustrate a process for the preparation of the two isomers of Compound A using just one enantiomer of phenylglycinol to generate two diastereoisomers of the ester, which are separated and then reduced to diols.

## Example 3

(R)-(-)-4-Hydroxy-α[1][[[6-(4-phenylbutoxy)hexyl]amino]methyl]-1,3-benzenedimethanol

(i) [R-(R*,R*)] Methyl 5-[1-hydroxy-2-[(2-hydroxy-1-phenylethyl)[6-(4-phenylbutoxy)hexyl]amino]-2-(phenylmethoxy)benzoate

A mixture of methyl 5-bromoacetyl-2-(phenylmethoxy)benzoate (34.5g) and (R)-β-[[6-(4-phenylbutoxy)-hexyl]amino]benzeneethanol (the product of Example 1, part (i), 35.0g,) in acetonitrile (500ml) containing diisopropylethylamine (18.7g) was heated under reflux for 4.5 hours. The very dark brown solution was evaporated under reduced pressure, dimethoxyethane (500ml) added and the suspension filtered. Methanol (50ml) was added to the filtrate followed by the portionwise addition of sodium borohydride (9.0g). The mixture was stirred at room temperature for 18 hours followed by the cautious addition of pH6.5 buffer and then the mixture was poured into water (400ml) and ethyl acetate (400ml). After separating the phases, the aqueous phase was extracted with ethyl acetate (200ml). The combined organic extracts were washed with brine (600ml), dried and evaporated under reduced pressure. The residue was chromatographed on silica gel (Sorbsil C60; 3.3Kg) eluting with hexane:propan-2-ol:triethylamine: dichloromethane (92:2:5:10). Appropriate eluates were collected and evaporated under reduced pressure to give the title compound as a pale yellow oil (17.0g).
HPLC ratio of R:S = 96.7:3.3
n.m.r. δ ($CDCl_3$) 1.3-1.75 (12H,m), 2.32-2.85 (6H,m), 3.32-3.48 (4H,2xt), 3.78 (1H,m), 3.9 (3H,s), 4.04 (2H,m), 4.72 (1H,dd), 5.18 (2H,s), 6.99 (1H,d), 7.11-7.52 (16H,m), 7.78 (1H,d).

(ii) [R-(R*,R*)]-α[1]-[[(2-Hydroxy-1-phenylethyl)[6-(4-phenylbutoxy) hexyl]amino]methyl]-4-(phenylmethoxy)-benzene-1,3-dimethanol

A mixture of the product of part (i) (4.50g) and lithium borohydride (0.45g) in dimethoxyethane (90ml)

was heated under reflux for 22 hours. Phosphate buffer (pH6.5, 20ml) was added cautiously to the reaction mixture and stirred for 15 mins. Residual solid was dissolved by adding 2N hydrochloric acid (5ml), the solution was poured into water (100ml), basified with 2N sodium hydroxide and extracted with ethyl acetate (2x150ml). The combined organic phases were dried, filtered and the filtrate evaporated in vacuo to give a pale yellow oil (4.15g). Purification by flash column chromatography on silica (Sorbsil C60, 20-40mcms. diam. 9cm, 400g) eluting with hexane:propan-2-ol:triethylamine:dichloromethane (90:5:10:5) gave the title compound as a clear oil (2.64g).

HPLC ratio of R:S = 99.6:0.4 Chemical purity 96.5%.

n.m.r. δ ($CDCl_3$) 1.29-1.8 (12H,m), 2.33-2.9 (6H,m), 3.13 (1H,br.s), 3.35-3.5 (4H,2xt), 3.76 (1H,m), 4.03 (2H,m), 4.66-4.77 (3H,m), 5.11 (2H,s), 6.92 (1H,d), 7.1-7.45 (17H,m).

The product of part (ii) was deprotected by catalytic hydrogenation according to the method of Example 1, part (iii) to yield the title compound as a free base.

## Example 4

### (S)-(+)-4-Hydroxy-α[1]-[[[6-(4-phenylbutoxy)hexyl]amino]methyl]-1,3-benzenedimethanol, 1-hydroxy-2-naphthalenecarboxylate salt (1:1)

#### (i) [S-(R*,S*)] Methyl 5-[1-hydroxy-2-[(2-hydroxy-1-phenylethyl) [6-(4-phenylbutoxy)hexyl]amino]ethyl]-2-(phenylmethoxy)benzoate

The title compound was separated from the resultant products of the preparation of Example 3, part (i) by chromatography on silica gel (Sorbsil 60; 3.3kg) eluting with hexane:propan-2-ol:triethylamine: dichloromethane (92:2:5:10). Appropriate eluates were collected and evaporated under reduced pressure to give the title compound as a yellow oil (23.8g).

HPLC ratio of R:S = 97.2:2.8

n.m.r. δ ($CDCl_3$) 1.15-1.76 (12H,m), 2.31-2.9 (6H,m), 3.32-3.45 (4H,2xt), 3.8-4.0 (6H,m), 4.64 (1H,dd), 5.18 (2H,s), 6.99 (1H,d), 7.12-7.53 (16H,m), 7.78 (1H,d).

#### (ii) [S-(R*,S*)]-α[1]-[[(2-Hydroxy-1-phenylethyl)[6-(4-phenylbutoxy) hexyl]amino]methyl]-4-(phenylmethoxy)-benzene-1,3-dimethanol

A mixture of the product of step (i) (24.9g) and lithium borohydride (2.5g) in dimethoxyethane (500ml) was heated under reflux for 18 hours. To the cooled reaction mixture was added pH6.5 buffer (50ml) followed by water (200ml). The mixture was poured into ethyl acetate (200ml). The residue remaining in the flask was dissolved in 2N hydrochloric acid (20ml) and the solution was poured into the mixture. The phases were separated and the aqueous phase was extracted with ethyl acetate (100ml). The combined organic extracts were dried and evaporated under reduced pressure. The residue was chromatographed on silica gel (Sorbsil C60) eluting with hexane:propan-2-ol:triethylamine:dichloromethane (90:5:5:10). Appropriate eluates were collected and evaporated under reduced pressure to give the title compound as a colourless oil (17.1g)

HPLC ratio of S:R = 96.7:3.3 chemical purity 98%.

This product was combined with additional product (2.7g) prepared in an identical manner and the oil was chromatographed on silica gel (Sorbsil C60; 20.4μ; 1.1Kg) eluting with hexane:propan-2-ol: triethylamine:dichloromethane (135:5:5:10). Appropriate eluates were collected and evaporated under reduced pressure to give the title compound as a colourless oil (16.5g).

HPLC ratio of S:R = 98:2 Chemical purity 99.4%

n.m.r. δ ($CDCl_3$) 1.12-1.76 (12H,m), 2.28-2.88 (6H,m), 3.32-3.46 (4H,2xt), 3.77 (1H,m), 3.85-3.98 (2H,m), 4.65 (1H,dd), 4.72 (2H,br.d), 5.09 (2H,s), 6.91 (1H,d), 7.11-7.45 (17H,m).

#### (iii) (S)-(+)-4-Hydroxy-α[1]-[[[6-(4-phenylbutoxy)hexyl]amino]methyl]-1,3-benzenedimethanol, 1-hydroxy-2-naphthoate salt (1:1)

A solution of the product of step (ii) (15.9g) in ethanol (160ml) was added to a suspension of pre-reduced 10% palladised charcoal (wet paste; 1.6g) in ethanol (40ml). The mixture was stirred under an atmosphere of hydrogen until uptake (1,200ml) ceased. The suspension was filtered through a pad of "hyflo" and the filtrate was evaporated under reduced pressure. The residue was dissolved in diethyl ether (200ml) and a solution of 1-hydroxy-2-naphthoic acid (4.77g) in ether (100ml) was added dropwise. After being stirred for 30 minutes the solid was collected, washed with ether (100ml) followed by hexane (200ml). The material was dried in vacuo yielding the title compound as a white powder (12.51g).

n.m.r. $\delta$ (DMSO) 1.23-1.69 (12H,m), 2.58 (2H,t), 2.8-3.03 (4H,m), 3.34 (4H,2xt), 4.51 (2H,s), 4.77 (1H,dd), 5.03 (1H,br.s), 6.78 (1H,d), 6.96-7.51 (10H,m), 7.68-7.79 (2H,m), 8.22 (1H,dd).

HPLC Hypersil ODS 5$\mu$ Solution A:acetonitrile (65:35) adjusted to pH 5.7. Retention time = 5 minutes. (Solution A = 1M ammonium acetate:water (200:450) containing 0.5g tetrabutyl ammonium hydrogen sulphate).

## Claims

1. The compound (R)-(-)-4-hydroxy-$\alpha^1$-[[[6-(4-phenylbutoxy)hexyl] amino]methyl]-1,3-benzenedimethanol, or a physiologically acceptable salt thereof.

2. The compound according to claim 1 or a physiologically acceptable salt thereof for the manufacture of a medicament for the treatment, relief or prevention of a condition which may be ameliorated by a compound having selective $\beta_2$-adrenoreceptor stimulant activity.

3. A pharmaceutical formulation comprising the compound according to claim 1 or a physiologically acceptable salt thereof together with a physiologically acceptable carrier or excipient.

4. A method for the preparation of the compound according to claim 1 or a physiologically acceptable salt thereof, which comprises deprotection of a compound of formula (I)

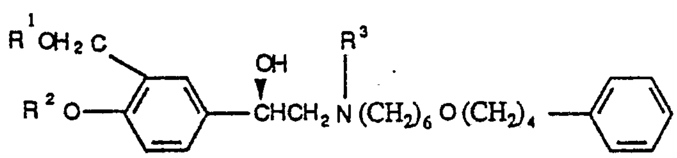

(I)

wherein $R^1$, $R^2$ and $R^3$ each independently represents a hydrogen atom or a protecting group with the proviso that at least one of them represents a protecting group; followed if desired by the conversion of the resulting compound or a salt thereof into a physiologically acceptable salt thereof.

5. A method according to claim 4 wherein $R^3$ is an (R)-2-hydroxy-1-phenylethyl group.

Claims for the following Contracting States: ES, GR

1. A method for the preparation of the compound (R)-(-)-4-hydroxy-$\alpha^1$-[[[6-(4-phenylbutoxy)hexyl] amino]-methyl]-1,3-benzenedimethanol or a physiologically acceptable salt thereof, which comprises deprotection of a compound of formula (I)

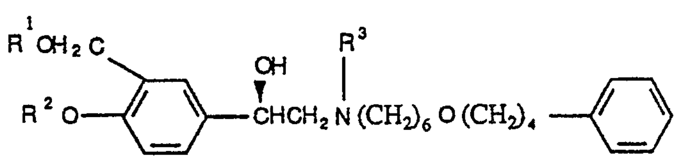

(I)

wherein $R^1$, $R^2$ and $R^3$ each independently represents a hydrogen atom or a protecting group with the proviso that at least one of them represents a protecting group; followed if desired by the conversion of the resulting compound or a salt thereof into a physiologically acceptable salt thereof.

2. A method according to claim 1 wherein $R^3$ is an (R)-2-hydroxy-1-phenylethyl group.